Europäisches Patentamt

European Patent Office

Office européen des brevets

(11) Publication number: **0 226 720**
A1

## (12) EUROPEAN PATENT APPLICATION

(21) Application number: 86112669.6

(22) Date of filing: 13.09.86

(51) Int. Cl.⁴: **A61M 1/34** , F04B 43/12 , F16K 15/16

(30) Priority: 28.10.85 SE 8505075

(43) Date of publication of application:
01.07.87 Bulletin 87/27

(84) Designated Contracting States:
BE CH DE FR GB IT LI NL

(71) Applicant: **GAMBRO AB**
**Post Box 10101**
**S-220 10 Lund(SE)**

(72) Inventor: **Aall-Flood, Jörgen**
**Banvallsvägen 3A**
**S-231 00 Trelleborg(SE)**
Inventor: **Carlsson, Per-Olof**
**Morkullevägen 11**
**S-280 10 Sösdala(SE)**

(74) Representative: **Boberg, Nils Gunnar Erik**
**Gambro AB Patent Department Box 10101**
**S-220 10 Lund(SE)**

(54) An arrangement for the prevention of back flow during pumping and a blood filtering system comprising such an arrangement.

(57) An arrangement for the prevention of back flow during pumping of a fluid through a duct comprising an elastic pump segment (19c) included in a peristaltic pump (19), **characterized** by a check valve (100) arranged directly behind the pump segment (19c) and a blood filtering system comprising such an arrangement.

The arrangement and the system in accordance with the invention are intended in particular to be used in connection with haemofiltration or plasmafers with supply of replacement fluid instead of the filtrate withdrawn.

Fig. 2

# AN ARRANGEMENT FOR THE PREVENTION OF BACK FLOW DURING PUMPING AND A BLOOD FILTERING SYSTEM COMPRISING SUCH AN ARRANGEMENT

## TECHNICAL FIELD

The present invention relates to an arrangement for the prevention of back flow during pumping of a fluid through a duct comprising an elastic pump segment included in a peristaltic pump.

An arrangement in accordance with the invention can be used in many connections. It is intended in particular, though, to form part of a blood filtering system which moreover comprises means for the withdrawal of blood from the patient or some other source, and a filter, with the help of which a filtrate can be withdrawn from the blood, and suitable means for the return of the remainder of the blood to the source together with any replacement fluid, a measuring device being provided for the measurement of the amount of filtrate withdrawn.

The said system may be constituted by one such intended for haemofiltration or plasmafers with supply of replacement fluid instead of the filtrate withdrawn. It will be clear, however, to those versed in the art that the invention can also be applied in other connections, e g in conjunction with haemodialysis, when the dialysis is combined with the withdrawal of an ultrafiltrate.

## BACKGROUND ART

Hemofiltration with preparation at the same time of replacement fluid is described, for example, in the Euroean patent applications published under No EP 0 042 939 and EP 0 087 171 respectively.

Plasmafers differ from haemofiltration principally in that they require a slightly more permeable membrane material for the filtering out also of larger molecules.

Examples of a suitable haemofiltration membrane are described in the European patent application published under No EP 0 046 816. In the same manner a suitable membrane for plasmafers is described in the European patent application published under No EP 0 044 958. It is clear, however, to those versed in the art that other membranes too can be used in connection with the realization of the present invention.

When it is intended in conjunction with, for example, hemofiltration or plasmafers to measure the amounts of filtrate withdrawn it has been found difficult to carry out exact measurements owing to the varying pressure conditions and the consequently varying conditions of flow. Special problems were encountered on application of the peristltic pumps normally used, since they act from the outside upon the flexible blood tubes in a pulsating manner.

The abovementioned problem has been largely solved with the help of a system which is described in more detail in the European patent application 85.106708.2. This was achieved here with the help of a pressure equalizing device for the equalizing ot the pressure in the filtrate flow passed through the measuring device. In this way the certainty of the measurement was improved substantially, though still not wholly satisfactory. It was suspected that the residual uncertainty is due to the microbubbles formed during the pumping which pass the pressure equalizing device designed as an air separating device.

In accordance with a preferred embodiment of the present invention a pressure equalizing device comprising a gaspermeable but liquid-tight membrane is used. Reference is made in this connection also to the US patent 4 198 971 which describes a drip chamber of a similar design.

## DISCLOSURE OF INVENTION

In its most general form the invention relates to an arrangement for the prevention of back flow during pumping of a fluid through a duct comprising an elastic pump segment included in a peristaltic pump. The arrangement is characterized by a check valve arranged directly behind the pump segment.

It is obvious to those versed in the art that in peristaltic pumps in general too it can be of interest to prevent back flow, e g in the pumping of blood where the blood can be damaged in the said back flow. Special advantages are gained, though, if the arrangement in accordance with the invention is used for a blood filtering system of the abovementioned type. It may be adapted so as to prevent back flow in the filtrate duct between a peristaltic pump for the pumping of the filtrate and a pressure equalizing device which in turn is arranged upstream of the measuring device included in the system. With the help of such a combination it has proved possible effectively to prevent back flow and microbubbles brought about by such a flow. The result has been confirmed with the help of areometer measurement.

Preferably the said pressure equalizing device is constituted of a drip chamber which is adapted to serve as an air separating device, the said arrangement for preventing backflow hindering effectively the formation of microbubbles, at the same time as the drip chamber separates larger air bubbles normally occurring in the filtrate.

In a preferred embodiment of the object of the invention the said check valve consists of an appropriately tubular body, joined at both its ends to a plastic pad with an inlet and an outlet, with the inlet opening between two thin flexible plastic films arranged inside the plastic pad. The plastic films are appropriately of elongated shape and are arranged face to face joined together along the periphery, with the said inlet opening at the one end, and a hole being provided in at least one of the plastic films at the other end. This hole is appropriately of triangular or other polygonal shape. The advantage of such a design is that it has proved to prevent effectively back flow of the filtrate in the filtrate duct, at the same time as it can be manufactured so inexpensively that, like the rest of the duct system, it need be used only once and can be discarded after this use.

## BRIEF DESCRIPTION OF DRAWINGS

Fig 1 shows in the form of a block diagram a preferred, substantially complete, system in accordance with the invention.

Fig 2 shows a preferred embodiment of some components included in the system according to Fig 1.

## BEST MODE OF CARRYING OUT THE INVENTION

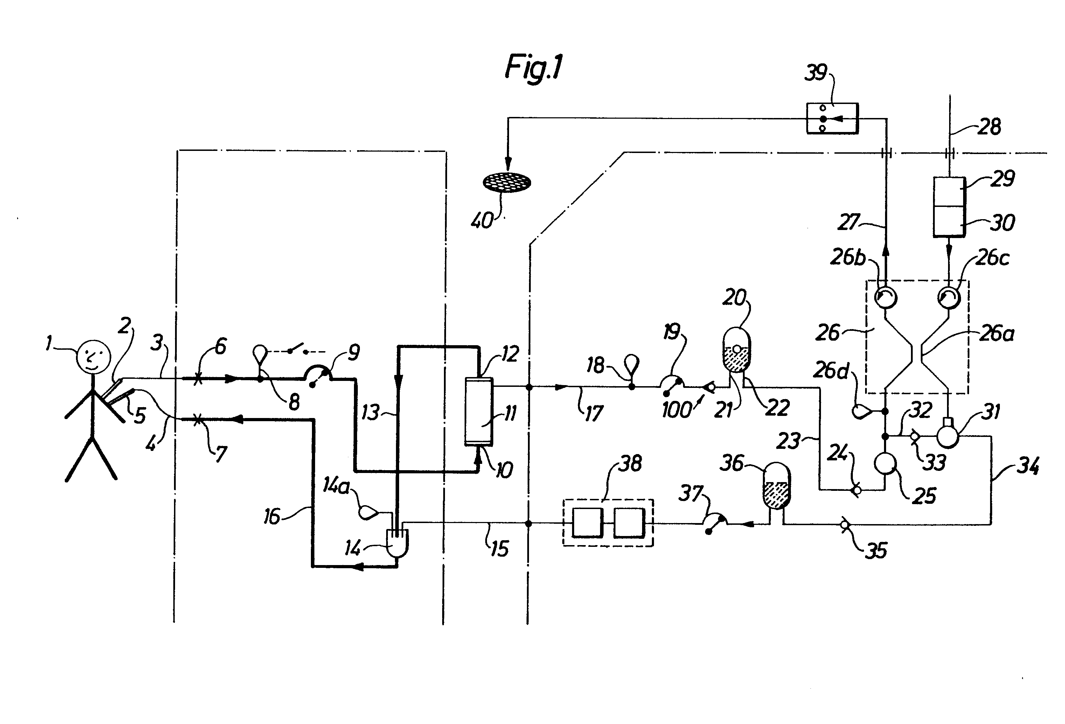

In the block diagram shown in Fig 1 a patient is designated 1. Blood is withdrawn from him by means of a cannula 2 and is conducted via a duct 3 into a blood treatment system. The blood is returned thereafter to the patient via a duct 4 and a cannula 5. For reasons of safety the ducts can be shut off and opened respectively with the help of clamps 6 and 7. After the clamp 6 the blood passes an arterial pressure gauge 8 arranged upstream of a peristaltic pump 9 by means of which the blood circulation is brought about. From the pump 9 the blood is pressed via an inlet 10 into a filter 11 and out from this via an outlet 12.

The invention is intended to be applied in particular in conjunction with haemofiltration or plasmafer and the filter thus consists here of a haemofilter or a plasmafer filter respectively.

From the outlet 12 the blood is taken via a duct 13 to a drip chamber 14 which is coordinated with a venous pressure gauge 14a. A replacement fluid is also conducted to the drip chamber 14 via a duct 15. From the drip chamber the blood mixed with replacement fluid is then conducted via a duct 16, the clamp 7 and the cannula 5 back to the patient.

From the filter 11 filtrate is withdrawn via a duct 17, the pressure being measured by means of a pressure gauge 18. To suck the filtrate from the filter 11 a pump 19 is used which preferably is constituted in conventional manner of a perstaltic pump.

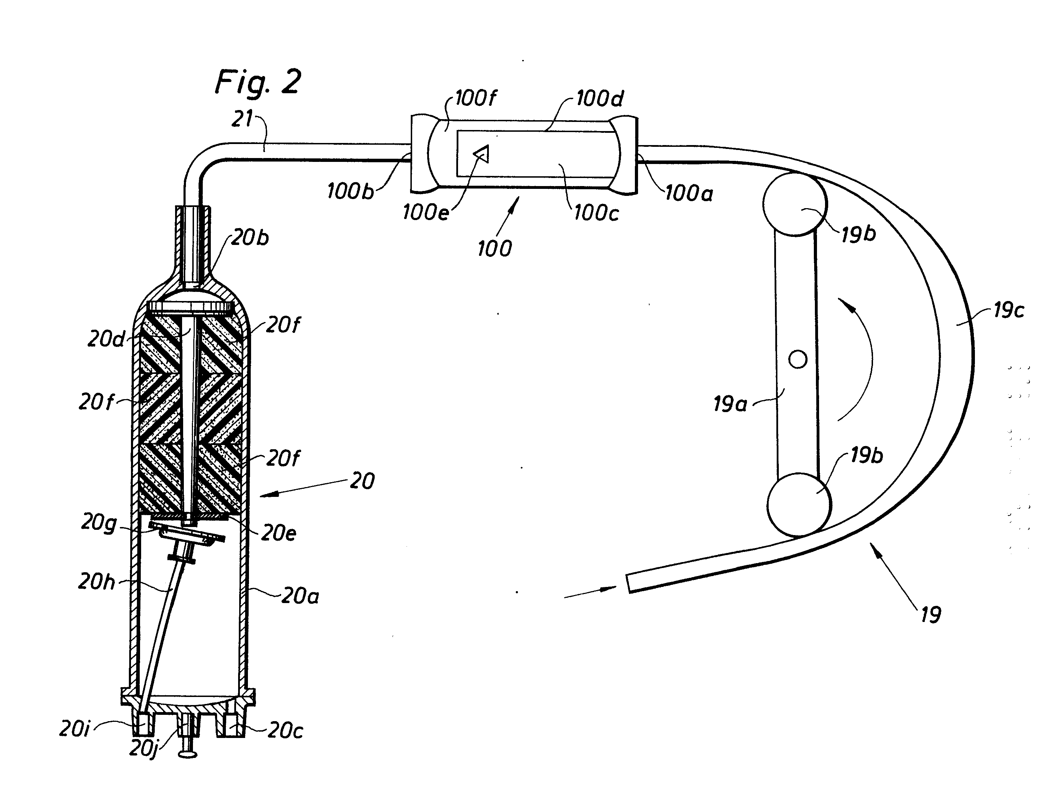

Up to this point the system corresponds to the European patent application 85.106708.2. Here follows, however, a check valve 100 inserted in accordance with the present invention which will be described in greater detail in connection with Fig 2 and which is intended to prevent effectively any back flow into the peristaltic pump 19. After the check valve 100 the filtrate then passes into a pressure equalizing device 20, which too will be described in greater detail in connection with Fig 2. The inlet to the pressure equalizing device is designated 21 and the outlet 22. The filtrate is then passed by means of a duct 23 via a check valve 24 and a suitable, controllable ultrafiltration valve 25 to a flow meter 26. This may be designed for example, in accordance with the European patent application, published under No EP 0 106 940, but can be substituted, of course, by other designs.

From the meter 26 the filtrate is taken via a duct 27 either to a collection point, if it is to be retained, or to a drain 40.

A replacement fluid prepared in a separate set-up is supplied to the meter 26 via a duct 28, a conductivity meter 29 and a temperature measuring instrument 30.

The meter 26 appropriately comprises a first part 26a with the help of which the difference between flows in the ducts 27 and 28 is measured. As a check, moreover, a measurement of the individual flows in parts 26b and 26c may also take place. For a possible adjustment of the measuring result in relation to the temperature the meter 26 may be coordinated, moreover, with a temperature measuring instrument 26d. From the meter 26 the replacement fluid is taken to a valve 31 which as a function of the values measured in the meters 29 and 30 conducts the replacement fluid either via a check valve 33 to a by-pass duct 32 and from there further to the dicharge pipe 27 or to a duct 34, and via a check valve 35, a pressure equalizing device 36, an infusion pump 37 and possibly a filter system 38 and the duct 15 to the drip chamber 14.

Numeral 39 in Fig 1 finally designates a valve which allows flow only when pressure exists in the system, but which immediately cuts the connection to the drain 40 should the pressure there be greater than in the remainder of the system. In such a case an air gap would be produced instead so that it would be impossible for fluid to pass from the drain 40 into the system.

PREFERRED DESIGNS OF COMPONENTS

In Fig 2 are shown some preferred designs of components and, more particularly, the pump 19, the check valve 100 and the pressure equalizing device 20. The pump 19 is shown schematically as a conventional peristaltic pump comprising a rotor 19a with two rollers 19b which are adapted to press a pump segment 19c against a holding-up element not shown. With the help of the pump the filtrate is forced into the check valve 100 via the inlet 100a and out of the check valve through an outlet 100b. The inlet 100a opens between two thin plastic films 100c which preferably are joined to each other along the whole, or parts, of the periphery 100d. From the space between the plastic films 100c the filtrate flows out through a triangular opening 100e into the padlike outer part 100f of the check valve 100 which it thus leaves through the outlet 100d. ·

From the check valve the filtrate flows via the duct 21 to the pressure equalizing device 20 which has been given a modified design as compared with that shown in Fig 1. More particularly the pressure equalizing device 20 comprises an outer shell 20a with an inlet 20b and an outlet 20c. Three annular defrothing elements 20f are suspended with the help of a rod 20d and a lock washer 20e. They may consist, for example, of a polyurethane foam coated with a special defrothing agent. As described in greater detail in the abovementioned European patent application 85.106708.2 the level is maintained constant in the device 20 with the help of a gas-permeable but liquid-tight membrane 20g which communicates via a duct 20h with an air outlet 20i. Numeral 20j finally designates an extra outlet which may be used, for example, for sampling or the like.

Naturally the invention is not limited simply to the embodiments described above, but can be varied within the scope of the following claims. Thus, for example, the components included in the system in accordance with the invention can be varied within wide limits in respect of form as well as of function. Compare also greater details described in the patents and patent applications mentioned above, whose contents hereby are included in the present description.

**Claims**

1. An arrangement for the prevention of back flow during pumping of a fluid through a duct comprising an elastic pump segment (19c) included in a peristaltic pump (19), **characterized** by a check valve (100) arranged preferably directly behind the pump segment.

2. A blood filtering system comprising an arrangement in accordance with claim 1 and further means (2) for the withdrawal of the blood from a patient or some other source (1), a filter (11) by means of which filtrate can be withdrawn from the blood, as well as suitable means (5) for the reurn of the remainder of the blood to the source (1) togeter with any replacement fluid, a measuring device - (26) being arranged for the measurement of the amount of filtrate withdrawn, **characterized** in that the said arrangement for the prevention of back flow is arranged in the filtrate duct between a peristaltic pump (19) for pumping the filtrate and a pressure equalizing device (20) which in turn is arranged upstream of the measurng device (26).

3. A system in accordance with claim 2, **characterized** in that the said pressure equalizing device (20) is constituted of a drip chamber, which is adapted to serve as an air separationg device, the said arrangement for the prevention of back flow hindering effectively the formation of microbubbles.

4. A system in accordance with claims 2 and 3, **characterized** in that the said check valve (100) consists of a plastic pad (100f) with an inlet f(100a) and an outlet (100b), with the inlet (100a) opening between two thin flexible plastic films (100c) arranged inside the plastic pad (100f).

5. A system in accordance with claim 4, **characterized** in that the plastic films (100c) are of elongated shape and are arranged face to face joined to each other along the whole or parts of the periphery (100d), with the said inlet (100a) opening at the one end and a hole (100ef) being provided in at least one of the plastic films at the other end of the same.

6. A system in accordance with claim 5, **characterized** in that the said hole (100e) is of polygonal, and in particular triagonal, shape.

Fig.1

## Fig. 2

0 226 720

| | **DOCUMENTS CONSIDERED TO BE RELEVANT** | | EP 86112669.6 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | US - A - 3 046 903 (G.W.JONES) | 1 | A 61 M 1/34 |
| Y | * Totality; especially fig. 6; column 3, lines 15-19,37-40; column 5, lines 52-58; claims 1,2,4 * | 2,3 | F 04 B 43/12<br>F 16 K 15/16 |
| | -- | | |
| D,P,Y | EP - A2 - 0 165 519 (GAMBRO LUNDIA) | 2,3 | |
| | * Totality; especially fig. 1,3-5; abstract; page 6, line 26 - page 8, line 1 * | | |
| P | & SE-A-8 403 244 (19-12-1985) | | |
| | -- | | |
| X | US - A - 3 649 138 (R.B.CLAY et al.) | 1 | |
| | * Totality; especially fig. 1; column 4, lines 20-25 * | | TECHNICAL FIELDS SEARCHED (Int. Cl.4) |
| | -- | | |
| X | US - A - 4 012 177 (S.S.YAKICH) | 1 | A 61 M 1/00 |
| A | * Totality * | 2 | F 04 B 43/00 |
| | -- | | F 16 K 15/00 |
| A | WO - A1 - 82/00 956 (M.DOYER) | 1,4 | |
| | * Fig. 1,2; page 4, lines 28-32, page 5, lines 32-34 * | | |
| | -- | | |
| D,A | EP - A1 - 0 042 939 (GAMBRO AB) | 2 | |
| | * Totality * | | |
| | -- | | |
| D,A | US - A - 4 198 971 (D.G.NOILES) | 3 | |
| | * Fig. 2; abstract * | | |
| | -- | | |

The present search report has been drawn up for all claims

| Place of search | Date of completion of the search | Examiner |
|---|---|---|
| VIENNA | 02-04-1987 | LUDWIG |

# EUROPEAN SEARCH REPORT

## DOCUMENTS CONSIDERED TO BE RELEVANT

EP 86112669.6

| Category | Citation of document with indication, where appropriate, of relevant passages | Relevant to claim | CLASSIFICATION OF THE APPLICATION (Int. Cl.4) |
|---|---|---|---|
| A | US - A - 4 240 630 (A.C.HOFFMAN) <br> * Fig. 8-10; column 6, lines 33-48 * | 4,5 | |
| A | US - A - 3 967 645 (R.G.GREGORY) <br> * Totality * | 4,5 | |

TECHNICAL FIELDS
SEARCHED (Int. Cl.4)

The present search report has been drawn up for all claims

| Place of search | Date of completion of the search | Examiner |
|---|---|---|
| VIENNA | 02-04-1987 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82